(19) Europäisches Patentamt
European Patent Office
Office européen des brevets

(11) Veröffentlichungsnummer: **0 328 029 B1**

# (12) EUROPÄISCHE PATENTSCHRIFT

(45) Veröffentlichungstag der Patentschrift: **08.12.93**

(51) Int. Cl.$^5$: **C07J 9/00**, C07J 43/00, C12Q 1/60

(21) Anmeldenummer: **89102028.1**

(22) Anmeldetag: **06.02.89**

## (54) Cholesterin-esterase-Farbsubstrat.

(30) Priorität: **08.02.88 DE 3803757**

(43) Veröffentlichungstag der Anmeldung:
**16.08.89 Patentblatt 89/33**

(45) Bekanntmachung des Hinweises auf die Patenterteilung:
**08.12.93 Patentblatt 93/49**

(84) Benannte Vertragsstaaten:
**AT BE CH DE ES FR GB GR IT LI LU NL SE**

(56) Entgegenhaltungen:
**GB-A- 2 053 924**
**US-A- 4 042 330**

**CHEMICAL PHARMACEUTICAL BULLETIN, Band 31, Nr. 1, Januar 1983, Tokyo (JP); S. KAMACHI et al., Seiten 162-167&NUM;**

(73) Patentinhaber: **BOEHRINGER MANNHEIM GMBH**

**D-68298 Mannheim(DE)**

(72) Erfinder: **Junius, Martina, Dr. rer. nat.**
**Eichenstrasse 4**
**D-8139 Bernried(DE)**
Erfinder: **Herrmann, Rupert, Dr. rer. nat.**
**In der Au 23**
**D-8120 Weilheim(DE)**
Erfinder: **Schmuck, Rainer, Dr. rer. nat.**
**Mitterweg 4**
**D-8121 Sindelsdorf(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3. 10/3.6/3.3. 1)

## Beschreibung

Cholesterinesterase (Cholesterinesterhydrolase EC 3.1.1.13) hydrolysiert emulgierte Cholesterinester langkettiger Fettsäuren an der Grenzfläche zwischen lipophiler und wäßriger Phase. Cholesterinesterase kommt im menschlichen Organismus vor, auch in Mikroorganismen. Besonders häufig wird die Cholesterinesterase als Forschungsreagenz und in der biochemischen Analytik verwendet. Insbesondere wird sie in vielen (diagnostischen) enzymatischen Tests als Hilfsenzym eingesetzt. Die Cholesterinesterase-Bestimmung ist daher für die klinische Chemie, aber auch für die Biochemie, die Mikrobiologie und die Lebensmittelchemie von erheblicher Bedeutung. Hierfür und bei der Herstellung und Isolierung des Enzyms besteht daher Bedarf an einem spezifischen, unkomplizierten Test.

Es sind bereits eine Reihe von Cholesterinesterase-Meßmethoden bekannt, die auf der Bestimmung der bei Cholesterinesterspaltung gebildeten Spaltprodukte beruhen. So kann bei radioaktiv markiertem Cholesterinester als Substrat die freigesetzte radioaktiv markierte Säure bestimmt werden (Biochim.Biophys.Acta 617, 446 (80)). Die Methode ist aufwendig, störanfällig und umständlich.

Bekannt ist die photometrische Bestimmung im UV. Freigesetztes Cholesterin wird mittels Cholesterinoxidase zu Cholestenon oxidiert, das anhand der Absorptionszunahme bei $\lambda$ = 240 nm gemessen werden kann (Bergmeyer, Methods of Enzymatic Analysis, 3th.Ed., Vol.II, S.168). Alternativ wird das bei dieser Oxidation gebildete Wasserstoffperoxid durch eine Farb-Reaktion bestimmt (Stähler, Med.Lab. 30, 29 (77)). Diese Methoden sind umständlich und ebenfalls störanfällig.

Verwendet werden auch fluoreszierende Substrate z.B. Pyrenbuttersäure- bzw. Pyrendecansäureester des Cholesterins. Nach Spaltung ist eine Änderung der Fluorenzenzausbeute feststellbar (Uster, Arch.Biochem.Biophys. 209, 385 (81)). Diese Methode ist jedoch stark störanfällig und die meisten Labors sind nicht mit Fluoreszenzmeßgeräten ausgestattet.

Bekannt ist auch, chromogene Substrate zur Quantifizierung der Cholesterinesterase einzusetzen, z.B. Fluoresceindilaurat oder Methylumbelliferylester (Meyer-Bertenrath, Enzyme Vol. 28, 336 (82); A.Negre, Biochim.Biophys. Acta 794, 89 (84)). Diese Substrate sind jedoch nicht spezifisch und werden auch von Esterasen gespalten.

Es besteht daher ein Bedarf an einem Farbtest, der mit einer einfachen Apparatur durchgeführt und direkt visuell kontrolliert werden kann.

Der Erfindung liegt nun die Aufgabe zugrunde, ein Substrat und einen Farbtest zur Bestimmung der Cholesterinesterase unter Verwendung dieses Substrats zu schaffen, der die Nachteile der bekannten Farbtests nicht aufweist, genaue Ergebnisse liefert, einfach in der Handhabung ist, hohe Empfindlichkeit besitzt und nur eine geringe lag-Phase aufweist, so daß die Adaptation an die verschiedenen Analysenautomatensysteme unschwierig ist.

Gelöst wird diese Aufgabe erfindungsgemäß durch ein Cholesterinesterasesubstrat der allgemeinen Formel I

$$X - \overset{\overset{\displaystyle O}{\|}}{C} - A - \overset{\overset{\displaystyle O}{\|}}{C} - Y - Chol \qquad\qquad I$$

worin

A eine Alkylen- oder Alkenylengruppe mit 1 bis 20 C-Atomen, Chol den 3-Cholesterinrest,

X den Rest einer aromatischen Hydroxy- oder Thiolverbindung und Y -S- oder -O- bedeuten.

Als 3-Cholesterinrest wird im Rahmen der vorliegenden Erfindung jeder über die 3-O-Gruppe verestert vorliegende unsubstituierte oder substituierte Steroidrest verstanden, der von der Cholesterinesterase hydrolysiert wird. Solche Steroidreste sind beispielsweise Cholesterin, Cholestanol, Dihydrocholesterin, $\beta$-Sitosterin, Stigmasterin oder Ergosterin.

Unter der Einwirkung der Cholesterinesterase wird der erfindungsgemäße Cholesterinester gespalten unter Freisetzung der dem Rest X entsprechenden aromatischen Hydroxy- oder Thiolverbindung, die man entweder direkt optisch bestimmt oder mit einem geeigneten Chromophor oder Fluorophor kuppelt und das Kupplungsprodukt mißt. Wenn die Kettenlänge von A 4 C-Atome übersteigt, wird vorzugsweise eine Esterase als Hilfsenzym zur Beschleunigung der Reaktion zugesetzt. Geeignete Esterasen sind z.B. Carboxylesterase oder Arylesterase.

Das erfindungsgemäße Cholesterinesterasesubstrat enthält weiter den Rest einer Dicarbonsäure COOH-A-COOH, in der A vorzugsweise 3 bis 7 C-Atome aufweist. Beispiele für Säuren, von denen sich A ableitet,

sind Malonsäure, Bernsteinsäure, Glutarsäure, Adipinsäure, Pimelinsäure, Korksäure, Azelainsäure, Sebacinsäure, Nonandicarbonsäure, Decandicarbonsäure und Undecandicarbonsäure. Die Säuren von Glutarsäure bis Azelainsäure, die A mit 3 bis 7 C-Atomen entsprechen, werden wie oben erwähnt, bevorzugt.

X ist der Rest einer aromatischen Hydroxy- oder Thiolverbindung, die ein Chromophor darstellt oder erst durch eine Folgereaktion in einen Farbstoff überführt werden kann. Typische Beispiele sind Phenol, Thiophenol, Naphthol, Thionaphthol und deren Derivate sowie die an sich chromogenen Verbindungen wie der Resorufin-, Chlorphenolrot-, Indoxyl- oder Thiofluoreszeinrest. Eine erschöpfende Aufzählung der geeigneten Hydroxy- oder Thiolverbindungen ist aufgrund ihrer großen Zahl nicht möglich, die direkt chromophoren oder in Chromophore überführbaren aromatischen Hydroxy- oder Thiolverbindungen sind dem Fachmann jedoch bekannt.

Die genannte Folgereaktion zum Farbstoff kann entweder durch eine direkte Kupplung (z.B. mit einem Diazoniumsalz wie 4-Chlor-2-methylbenzoldiazoniumsalz (Fast Red), 4-Benzamido-2-methoxy-5-methylbenzol-diazoniumsalz (Fast Violet), diazotierte Sulfanilsäure, 2,4- und 2,5-substituierte Phenyldiazoniumsalze z.B. 2,4-Dichlorphenyldiazonium-1,5-naphthalin-disulfonsäure) erfolgen oder durch eine oxidative Kupplung z.B. mit 4-Aminoantipyrin oder anderen Aminopyrazolonen (wie Trimethylaminopyrazolon, Diaminoantipyrin) oder MBTHS (3-Methyl-2-benzothiazolinon-hydrazon-6-sulfonsäure).

Bevorzugt sind Chromophore, die eine geringe Polarität aufweisen und lipophil sind. Der lipophile Charakter der o.g. Chromophore kann durch geeignete Substitution, wie z.B. mit Alkylgruppen, positiv beeinflußt werden. Als geeignete Substituenten, insbesondere für den Resorufinrest, haben sich u.a. die Methyl-, Dimethyl- und Äthylgruppe sowie Brom erwiesen.

Die Herstellung der erfindungsgemäßen Cholesterinesterasesubstrate kann nach an sich bekannten Methoden erfolgen. So lassen sich z.B.aus Cholesterin und Dicarbonsäureanhydriden, die sich von Dicarbonsäuren der allgemeinen Formel

HOOC - A - COOH

ableiten, in der A die oben angegebene Bedeutung hat, in wasserfreiem Medium wie Chloroform/Pyridin die Halbester synthetisieren.

Geeignete Methoden zur Herstellung der Dicarbonsäureanhydride sind in Houben-Weyl-Müller "Methoden der organischen Chemie", Band IV/4, Seite 786 beschrieben.

Die Veresterung des so erhaltenen Halbesters mit der aromatischen Hydroxy- oder Thiolverbindung, von der sich der Rest X ableitet, kann beispielsweise durch direkte Umsetzung des Dicarbonsäuremonoesters mit dem aromatischen Alkohol oder Thiol in Gegenwart eines Wasser entziehenden Mittels wie Dicyclohexylcarbodiimid, durchgeführt werden. Alternativ wird der Dicarbonsäuremonoester zuerst in einen aktivierten Ester überführt, beispielsweise in den Hydroxysuccinimidester oder das Imidazolid und der aktivierte Ester wird dann mit dem aromatischen Alkohol oder Thiol umgesetzt.

Ebenso ist es auch möglich, zuerst einen Monoester der Dicarbonsäure mit dem aromatischen Alkohol oder Thiol herzustellen, beispielsweise den Adipinsäure-Mononitrophenylester oder Glutarsäuremonophenylester, und diesen dann mit dem Cholesterin zu verestern, beispielsweise über intermediäre Bildung eines Säurechlorids, -anhydrids oder aktivierten Esters. Die Herstellung der Dicarbonsäuremonoester mit dem aromatischen Alkohol oder Thiol kann beispielsweise aus Säureanhydrid und aromatischer Verbindung im Molverhältnis 1:1 oder aus aktivierter Dicarbonsäure und aromatischer Verbindung oder aus einem Dicarbonsäuremonoester mit leicht abspaltbarer Schutzgruppe und der aromatischen Verbindung erfolgen. Geeignete Methoden sind beispielsweise in Arch. Pharm. 287, 514 (1954) beschrieben.

Das erfindungsgemäße Verfahren zur optischen Bestimmung der Cholesterinesterase ist dadurch gekennzeichnet, daß man ein erfindungsgemäßes Cholesterinesterasesubstrat der Einwirkung der cholesterinesterasehaltigen Probe unterwirft und die Menge der freigesetzten aromatischen Hydroxy- oder Thiolverbindung direkt oder, nach Kopplung mit einem geeigneten Chromogen, die daraus gebildete Farbe optisch bestimmt.

Ein besonderes Merkmal dieses Verfahrens besteht darin, daß es in einer bevorzugten Ausführungsform keine Hilfsenzyme oder Esterasehemmstoffe erfordert, wie sie bei den bekannten Methoden vielfach benötigt werden. Derartige Zusätze sind nicht nur teuer, sondern vielfach auch wenig stabil.

Ein weiterer Gegenstand der Erfindung ist ein einfaches und gut haltbares Reagenz zur optischen Bestimmung der Cholesterinesterase, welches neben einem erfindungsgemäßen Cholesterinesterasesubstrat und Puffersubstanz auch ein Detergens, insbesondere ein nichtionogenes Detergens, einen chromogenen Kuppler oder/und ein Salz wie Natriumchlorid enthält. Außerdem kann das Reagenz zweckmäßig Konservierungsmittel oder/und Aktivator enthalten.

In einer bevorzugten Zusammensetzung enthält dieses Reagenz
0,05 bis 2 mg/ml Substrat,
10 bis 50 µl Detergens,
10 bis 50 µl Dioxan,
50 bis 500 mM Puffersubstanz,
jeweils bezogen auf gebrauchsfertige Lösung im Test.

Bevorzugt wird als Detergens Polyethylenglycol-monododecylester (Thesit ®).

Als Puffersubstanz eignen sich solche, welche in der Lage sind, im Rahmen des erfindungsgemäßen Reagenz einen pH-Wert zwischen 5,5 und 10,5 einzustellen. Der bevorzugte pH-Wertbereich liegt zwischen 6,5 und 7,8. Beispiele für geeignete Puffer sind Diethanolaminpuffer, Triethanolaminpuffer, Tris- Puffer und Goodpuffer, wie Hepes-Puffer (gut geeignet zum Zusatz vor der Lyophilisation), Taps-Puffer, CHES- Puffer (2-(Cyclohexylamino)-ethansulfonsäure) und Bicine. Besonders bevorzugt wird Kalium-Phosphat-Puffer. Die bevorzugte Puffersubstanzmenge liegt zwischen 50 und 500 mM.

Liegt das erfindungsgemäße Reagenz in trockener oder konzentrierter, zur Verdünnung auf die endgültige Zusammensetzung bestimmter Form vor, so enthält es die genannten Substanzen in entsprechenden Mengenverhältnissen, sowie vorzugsweise Schutzkolloid.

Als Schutzkolloid kommen die dem Fachmann hierfür bekannten Substanzen in Betracht, wie Polyhydroxyverbindungen, Serumalbumin, Polyvinylpyrrolidon, feste Polyethylenoxide und dergleichen. Bevorzugt werden Polyhydroxyverbindungen, insbesondere monomere oder polymere Pentose oder Hexose mit 1 bis 10 Pentose- bzw. Hexose-Einheiten im Molekül oder/und bei Zimmertemperatur fester Polyethylenglykol. Bevorzugte Beispiele von geeigneten Polyhydroxyverbindungen sind Mannit und ähnliche Zuckeralkohole, Oligosaccharid der Glucose, Mannose, Maltoheptaose, Polyethylenglykol mit einem mittleren Molekulargewicht zwischen 3500 und 7000 u. ä. Andere verwendbare Schutzkolloide sind z. B. Aminosäuren, wie Alanin, Pflanzengummis, wie Gummi arabicum usw. Die bevorzugte Menge an Schutzkolloid bzw. einer Mischung von Schutzkolloiden beträgt 20 bis 90 Gew.-%. Als besonders geeignet erwies sich eine Mischung von Zuckeralkohol und Polyalkylenglykol.

Das erfindungsgemäße Reagenz kann auch auf einem geeigneten Trägermaterial imprägniert vorliegen. In betracht kommt sowohl ein saugfähiges Trägermaterial, als auch ein quellfähiges, lösliches filmbildendes Trägermaterial. In dieser Form ermöglicht das erfindungsgemäße Reagenz die Herstellung von Teststreifen, welche direkt visuell oder mittels geeigneter Meßgeräte ausgewertet werden können.

Der erfindungsgemäße Farbtest zur Bestimmung der Cholesterinesterase liefert sehr genaue Ergebnisse bei hoher Empfindlichkeit. Er ist sehr einfach in der Handhabung und eignet sich sogar für Teststreifen. Da er nur eine sehr geringe oder gar keine lag-Phase aufweist, kann er auf die verschiedenen Analysenautomatensysteme ohne weiteres adaptiert werden.

Die Bestimmung selbst kann sowohl als Endpunktbestimmung als auch kinetisch durchgeführt werden. Gegenüber vielen bekannten Verfahren liegt in der kinetischen Durchführbarkeit der Vorteil, daß weder ein Abstoppen, noch ein Ausschütteln des gebildeten Reaktionsproduktes durchgeführt werden muß.

Die folgenden Beispiele und die Abbildung erläutern die Erfindung weiter.

Figur 1 zeigt den Vergleich der Eichkurven (Δ E/min gegen Substratkonzentration mg/ml) für die Cholesterin-Oleat-Methode und das Verfahren unter Verwendung eines erfindungsgemäßen Substrats (Cholesterin-3-glutarsäure-resorufinester).

## Beispiel 1

### Cholesterin-glutarsäure-resorufinester

a) Cholesterin-glutarsäuremonoester

19,5 g Cholesterin werden in 150 ml Chloroform gelöst, mit 16 ml Pyridin, 11,5 g Glutarsäureanhydrid und einer Spatelspitze Dimethylaminopyridin versetzt. Nach 8-stündigem Erhitzen auf 65 °C wurde abgekühlt, dann mit 200 ml Chloroform verdünnt und zwei Mal mit 2N Salzsäure, 1 Mal mit Wasser gewaschen. Die organische Phase wird über Natriumsulfat getrocknet, eingeengt und an Kieselgel (Laufmittel: Essigester/Petrolether 1:1) chromatographiert.
Ausbeute: 6 g
Smp: 125 °C
$^1$H-NMR($CDCl_3$): . δ [ppm] : 0,68 (s,3H); 0,83 (s,3H); 0,90 (s,3H); 1,02 (s,3H); 0,95 - 1,95 (m,31H); 2,0 (m,2H) 2,4 (m,4H); 4,6 (m,1H); 5,37 (d,1H).

b) Cholesterin-glutarsäurechlorid

2,55 g 1a) werden in 20 ml Chloroform gelöst und unter Eiskühlung mit 2,2 ml Oxalylchlorid versetzt. Anschließend wird 1 Tropfen Dimethylformamid zugegeben und 8 h bei Raumtemperatur gerührt. Das Lösungsmittel wird abgezogen und der Rückstand roh weiterverarbeitet.

c) Cholesterin-glutarsäure-resorufinester

1,1 g Resorufin werden in 40 ml Chloroform aufgeschlämmt, mit 0,75 ml Diazabicycloundecen und 0,1 mg Dimethylaminopyridin versetzt. Bei Eiskühlung wird langsam eine Lösung von 1b) in 20 ml Chloroform getropft und anschliessend 8 h bei Raumtemperatur gerührt. Dann wird mit 50 ml Chloroform verdünnt und mit 50 ml 2N Salzsäure geschüttelt. Die organische Phase wird nach Trocknung über Natriumsulfat eingeengt und der Rückstand an Kieselgel (Laufmittel: Essigester/Petrolether 1:3) chromatographiert.
Ausbeute: 200 mg
DC: $R_f$ = 0,6 (Kieselgel; Essigester/Petrolether 1:2)
$^1$H-NMR($CDCl_3$): . δ[ppm] : 0,68 (s,3H); 0,83 (s,3H); 0,90 (s,3H); 1,02 (s,3H); 0,95 - 2,6 (m,35H); 2,70 (t,2H); 4,62 (m,1H); 5,37 (d,1H); 6,33 (d,1H); 6,86 (dd,1H); 7,13 (dd,1H); 7,17 (s,1H); 7,43 (d,1H); 7,80 (d,1H).

**Beispiel 2**

2 mg Cholesterin-glutarsäureresorufinester werden in 0,5 ml Dioxan und 0,5 ml Thesit® gelöst.
Zur Messung werden 850 µl 0,1 M Kalium-Phosphat-Puffer pH 6,8 und 50 µl der oben beschriebenen Substratlösung in einer Küvette gut durchgemischt. Nach Zugabe von 100 µl Probe (Enzymlösung) wird ΔE/min photometrisch bei λ = 571 nm bestimmt (Temperatur = 25 °C).
Bei Auswertung über einen Standard bekannter Cholesterinesteraseaktivität wird die Cholesterinesteraseaktivität der Probe wie folgt berechnet

$$\text{Aktivität}_{(\text{Probe})} = \frac{\text{Aktivität}_{(\text{Standard})} \cdot \Delta E/\text{min (Probe)}}{\Delta E/\text{min (Standard)}}$$

Eine Berechnung der Cholesterinesteraseaktivität der Probe ist auch nach folgender Formel möglich:

$$\text{Aktivität (Probe) } [U/l] = 1000 \cdot \frac{V_{ges}}{\varepsilon \cdot V_{Probe} \cdot d} \cdot \Delta E/\text{min}$$

| | |
|---|---|
| $V_{ges}$ : | Gesamtvolumen des Testansatzes [$cm^3$] |
| $V_{Probe}$ : | Volumen der Probe [$cm^3$] |
| ε : | Extinktionskoeefizient des Chromogens bei 571 nm |
| d : | Schichtdicke der Küvette [cm] |
| ΔE/min : | Extinktionsänderung pro Minute bei 571 nm |

Bei den genannten Reaktionsbedingungen beträgt der Extinktionskoeffizient $\epsilon = 60{,}00 \cdot cm^2 \cdot \mu mol^{-1}$.

**Beispiel 3**

Zum Vergleich wurde der Cholesterinesterase-Test mit Cholesterinoleat als Substrat durchgeführt (Bergmeyer, Methods of Enzymatic Analysis, 3th.Ed., Vol.II, S. 168) und mit dem Farbtest gemäß Beispiel 2 verglichen und der Korrelationskoeffizient ermittelt. Die Ergebnisse zeigen Tabelle I und Figur 1.

TABELLE I

| Cholesterin-Oleat | | Cholesterin-Glutarsäure-Resorufinester | |
|---|---|---|---|
| Konz.(mg/ml) | ΔE/min | Konz.(mg/ml) | ΔE/min |
| 10,0 | 0,072 | 10,0 | 0,071 |
| 6,6 | 0,045 | | |
| 5,0 | 0,036 | 5,0 | 0,033 |
| 2,0 | 0,015 | 2,0 | 0,015 |
| 1,3 | 0,011 | 1,3 | 0,013 |
| 1,0 | 0,007 | 1,0 | 0,009 |
| Leerwert: | 0,002 | Leerwert: | 0,0027 |
| Korrelationskoeffizient: 0,998 | | | |

**Beispiel 4**

Cholesterin-azelainsäure-resorufinester

a) Cholesterin-azelainsäuremonoester

Herstellung analog Beispiel 1a) aus 5,68 g Cholesterin, 7,5 g Azelainsäureanhydrid und 50 ml Chloroform
Ausbeute: 2,8 g
DC: $R_f$ = 0,60 (Kieselgel; Chloroform/Methanol 9:1)

b) Cholesterin-azelainsäurechlorid

Herstellung analog Beispiel 1b) aus 1,26 g 4a), und 1 ml Oxalylchlorid.

c) Cholesterin-azelainsäure-resorufinester

Herstellung analog Beispiel 1c) aus 4b), 0,72 g Resorufin und 0,52 ml Diazabicycloundecen
Ausbeute: 210 mg
DC: $R_f$ = 0,41 (Kieselgel; Essigester/Petrolether 2:3)

**Beispiel 5**

3β-Cholestanyl-glutarsäure-resorufinester

a) 3β-Cholestanyl-glutarsäuremonoester

Herstellung analog Beispiel 1a) aus 19,4 g 5 α-Cholestan-3β-ol, 23 g Glutarsäureanhydrid und 150 ml Chloroform.
Ausbeute: 22 g
DC: $R_f$ = 0,63 (Kieselgel; Chloroform/Methanol 8:2)

b) 3β-Cholestanyl-glutarsäurechlorid

Herstellung analog Beispiel 1b) aus 5 g 5a) und 4,5 ml Oxalylchlorid.

c) 3β-Cholestanyl-glutarsäure-resorufinester

Herstellung analog Beispiel 1c) aus 5b), 2,1 g Resorufin und 1,5 ml Diazabicycloundecen.
Ausbeute: 1,2 g
DC: $R_f$ = 0,29 (Kieselgel; Essigester/Petrolether 2:3)

## Patentansprüche

**1.** Cholesterinesterasesubstrat der allgemeinen Formel I

$$X - \overset{\overset{O}{\|}}{C} - A - \overset{\overset{O}{\|}}{C} - Y - Chol \qquad I$$

worin
A eine Alkylen- oder Alkenylengruppe mit 1 bis 20 C-Atomen, Chol einen über die 3-O-Gruppe verestert vorliegenden unsubstituierten oder substituierten und von der Cholesterinesterase hydrolisierbaren Steroidrest,
X den Rest einer aromatischen Hydroxy- oder Thiolverbindung und Y -S- oder -O- bedeuten.

**2.** Cholesterinesterasesubstrat nach Anspruch 1,
**dadurch gekennzeichnet,**
daß A 3 bis 7 C-Atome aufweist.

**3.** Cholesterinesterasesubstrat nach Anspruch 1 oder 2,
**dadurch gekennzeichnet,**
daß X ein gegebenenfalls substituierter Resorufin-, ein Chlorphenolrot-, Indoxyl-, Naphthol-, Thiophenol-, Thiofluoreszein- oder Phenolrest ist.

**4.** Cholesterinesterasesubstrat nach Anspruch 3,
**dadurch gekennzeichnet,**
daß X ein Methyl-, Dimethyl-, Ethyl- oder Bromsubstituierter Resorufinrest ist.

**5.** Cholesterin-3-glutarsäure-resorufinester.

**6.** Verfahren zur optischen Bestimmung der Cholesterinesterase,
**dadurch gekennzeichnet,**
daß man ein Substrat gemäß Anspruch 1 der Einwirkung der cholesterinesterasehaltigen Probe unterwirft und die Menge der freigesetzten aromatischen Hydroxy- oder Thiolverbindung direkt oder, nach Kopplung mit einem geeigneten Chromogen, die daraus gebildete Farbe optisch bestimmt.

**7.** Reagenz zur optischen Bestimmung der Cholesterinesterase,
**dadurch gekennzeichnet,**
daß es wenigstens ein Substrat nach einem der Ansprüche 1 bis 5, Puffersubstanz pH 5,5 bis 10,5, Detergens oder/und einen chromogenen Kuppler enthält.

**8.** Reagenz nach Anspruch 7,
**dadurch gekennzeichnet,**
daß es
0,05 bis 2 mg/ml Substrat,
10 bis 50 $\mu$l Detergens,
10 bis 50 $\mu$l Dioxan,
50 mM bis 500mM Puffersubstanz pH 6,5 bis 7,8 jeweils bezogen auf gebrauchsfertige Lösung im Test enthält.

**9.** Reagenz nach einem der Ansprüche 7 und 8,
**dadurch gekennzeichnet,**
daß es als chromogenen Kuppler ein kuppelbares Diazoniumsalz, 4-Aminoantipyrin oder MBTHS enthält.

**10.** Reagenz nach einem der Ansprüche 7 bis 9,
**dadurch gekennzeichnet,**
daß es auf einem Trägermaterial imprägniert vorliegt.

## Claims

**1.** Cholesterol esterase substrate of the general formula I

$$X - \overset{O}{\overset{\|}{C}} - A - \overset{O}{\overset{\|}{C}} - Y - Chol$$

wherein A signifies an alkylene or alkenylene group with 1 to 20 C-atoms, Chol is an unsubstituted or substituted steroid radical present esterified via the 3-O-group and hydrolysable by cholesterol esterase, X the radical of an aromatic hydroxy or thiol compound and Y -S- or -O-.

**2.** Cholesterol esterase substrate according to claim 1, characterised in that A has 3 to 7 C-atoms.

**3.** Cholesterol esterase substrate according to claim 1 or 2, characterised in that X is a possibly substituted resorufin, a chlorophenol red, indoxyl, naphthol, thiophenol, thiofluorescein or phenol radical.

**4.** Cholesterol esterase substrate according to claim 3, characterised in that X is a methyl-, dimethyl, ethyl- or bromine-substituted resorufin radical.

**5.** Cholesterol-3-glutaric acid resorufin ester.

**6.** Process for the optical determination of cholesterol esterase, characterised in that one subjects a substrate according to claim 1 to the action of the cholesterol esterase-containing sample and optically determines the amount of the liberated aromatic hydroxy or thiol compound directly or, after coupling with a suitable chromogen, the colour formed therefrom.

**7.** Reagent for the optical determination of cholesterol esterase, characterised in that it contains at least one substrate according to one of claims 1 to 5, buffer substance pH 5.5 to 10.5, detergent and/or a chromogenic coupler.

**8.** Reagent according to claim 7, characterised in that it contains:
0.05to 2 mg/ml of substrate,
10 to 50 µl of detergent,
10 to 50 µl of dioxane,
50 mM to 500 mM of buffer substance pH 6.5 to 7.8,
in each case referred to the solution ready to use in the test.

**9.** Reagent according to one of claims 7 and 8, characterised in that, as chromogenic coupler, it contains a couplable diazonium salt, 4-aminoantipyrine or MBTHS.

**10.** Reagent according to one of claims 7 to 9, characterised in that it is present impregnated on a carrier material.

## Revendications

**1.** Substrat de cholestérol-estérase de formule générale I

$$X - \overset{\overset{\displaystyle O}{\|}}{C} - A - \overset{\overset{\displaystyle O}{\|}}{C} - Y - Chol \qquad I$$

dans laquelle
A représente un groupe alkylène ou alcénylène de 1 à 20 atomes de carbone, Chol représente un reste stéroïde substitué ou non substitué, estérifié par l'intermédiaire du groupe 3-O et hydrolysable par la cholestérol-estérase, X représente le reste d'un composé hydroxylé ou thiolique aromatique et Y représente -S- ou -O-.

**2.** Substrat de cholestérol-estérase selon la revendication 1, caractérisé en ce que A comporte 3 à 7 atomes de carbone.

**3.** Substrat de cholestérol-estérase selon la revendication 1 ou 2, caractérisé en ce que X représente un reste résorufine éventuellement substitué, un reste rouge de chlorophénol, un reste indoxyle, un reste naphtol, un reste thiophénol, un reste thiofluorescéine ou un reste phénol.

**4.** Substrat de cholestérol-estérase selon la revendication 3, caractérisé en ce que X est un reste résorufine substitué par méthyle, diméthyle, éthyle ou le brome.

**5.** 3-cholestérylglutarate de résorufine.

**6.** Procédé de détermination optique de la cholestérolestérase, caractérisé en ce que l'on soumet un substrat selon la revendication 1 à l'action de l'échantillon contenant la cholestérol-estérase et on détermine la quantité de composé hydroxylé ou thiolique aromatique libéré directement ou bien, après couplage avec un chromogène approprié, on détermine optiquement la couleur qui en résulte.

**7.** Réactif pour la détermination optique de la cholestérol-estérase, caractérisé en ce qu'il contient au moins un substrat selon l'une des revendications 1 à 5, une substance tampon pH 5,5 à 10,5, un détergent et/ou un coupleur chromogène.

**8.** Réactif selon la revendication 7, caractérisé en ce qu'il contient
0,05 à 2 mg/ml de substrat,
10 à 50 µl de détergent,
10 à 50 µl de dioxanne,
50 mM à 500 mM de substance tampon pH 6,5 à 7,8
rapportés chacun à la solution prête à l'emploi dans le test.

**9.** Réactif selon l'une des revendications 7 et 8, caractérisé en ce qu'il contient comme coupleur chromogène un sel de diazonium capable de couplage, la 4-aminoantipyrine ou MBTHS.

**10.** Réactif selon l'une des revendications 7 à 9, caractérisé en ce qu'il est sous forme imprégnée sur un matériau support.

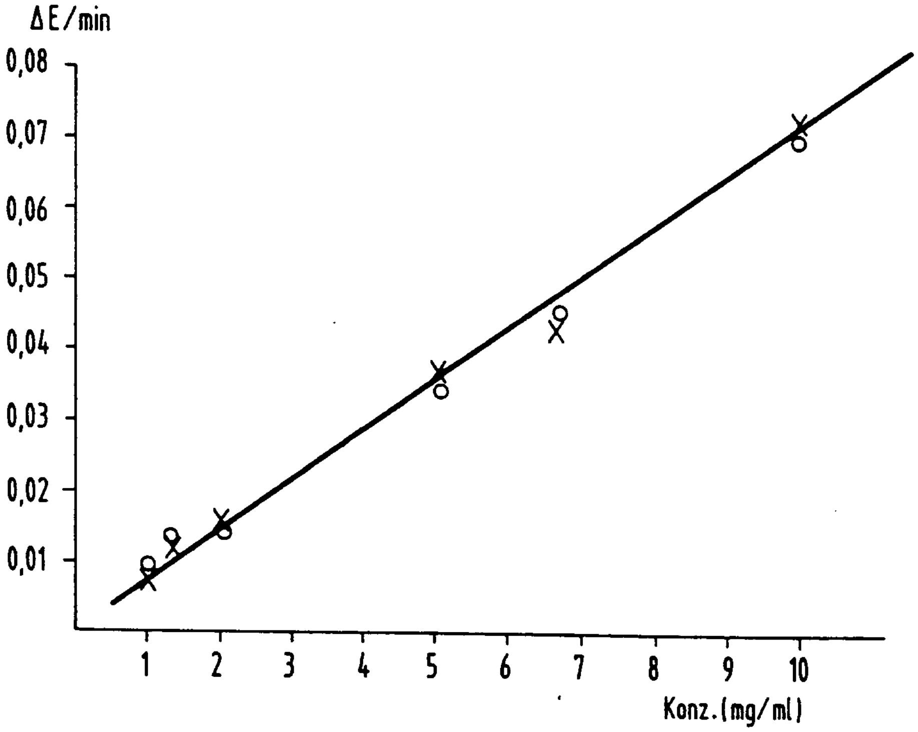


X = Test mit Cholesterin-Oleat
o = Test mit Cholesterin-glutarsäure-resorufinester